# EUROPEAN PATENT SPECIFICATION

(11) **EP 0 446 305 B1**
(45) Date of publication and mention of the grant of the patent: **08.05.1996**
(21) Application number: 90901336.9
(22) Date of filing: 05.12.1989
(51) Int. Cl.: C12Q 1/68

(54) **SYSTEMS FOR AMPLIFICATION/DETECTION OF TARGET NUCLEIC ACIDS**
SYSTEME FÜR AMPLIFIKATION/NACHWEIS VON NUKLEINSÄUREN
SYSTEMES D'AMPLIFICATION/DETECTION D'ACIDES NUCLEIQUES

(30) Priority: 05.12.1988 US 279817
(43) Date of publication of application: 18.09.1991
(73) Proprietor: THE SALK INSTITUTE FOR BIOLOGICAL STUDIES, La Jolla California 92037 (US)
(72) Inventor: CHU, Barbara, Chen, Fei, Del Mar, CA 92014 (US); JOYCE, Gerald, Francis, Encinitas, CA 92024 (US); ORGEL, Leslie, Eleazer, La Jolla, CA 92037 (US)
(74) Representative: Huber, Bernhard, Dipl.-Chem.
(86) International application number: US8905533
(87) International publication number: WO9006376

(56) References cited:
- WO-A-88/10315
- WO-A-90/14439
- US-A- 4 683 202
- US-A- 4 786 600
- JOURNAL OF MOLECULAR BIOLOGY, vol. 171, 1983; E.A. MIELE et al., pp. 281-295/
- BIOCHEMICAL & BIOPHYSICAL RESEARCH COMMUNICATIONS, vol. 65, no. 2, 1975; G. FEIX et al., pp. 503-509/
- NUCLEIC ACIDS RESEARCH, vol. 14, no. 14, 1986; B.C.F. CHU et al., pp. 5591-5603/
- BIOCHEMICAL & BIOPHYSICAL RESEARCH COMMUNICATIONS, vol. 70, no. 3, 1976; J. VOURNAKIS et al., pp. 774-782/
- BIOTECHNOLOGY, vol. 6, October 1988, New York, NY (US); P.M. LIZARDI et al., pp. 1197-1202/

## Description

### Field of the Invention

The present invention relates generally to advances in molecular biology and recombinant DNA technology.

More particularly, the invention is directed to the methods and means, including assays and test kits containing requisite reagents and means, for detecting in an in vitro or ex vivo setting the presence of target RNA species, and by deduction the corresponding polypeptide that the RNA species encodes, in a biological sample.

The present invention features combining in a novel manner, for use in nucleic acid probe hybridization systems for detection of target RNAs in biological samples, the advantages of target amplification with those of enzymatic production of detectable reporter molecules by using RNA-dependent RNA polymerases to make a copy of an RNA target that incorporates target sequence and is autocatalytically replicatable by the polymerase.

Among the applications in which the present invention finds use are in analyses of RNA sequences characteristic of a disease or pathogenic condition by in vitro or ex vivo nucleic acid probe hybridization assays of body fluids and tissues containing requisite target RNA.

### Background of the Invention

It is a goal in the nucleic acid probe art to detect various nucleic acid sequences in a biological sample, in which said sequences, as so-called target nucleic acid, are present, usually in small amounts relative to the wide variety of other nucleic acid species, including RNA, DNA or both, present in such a sample. Thus, it is desirable to be able to detect the RNA encoding polypeptides that may be associated with diseases or pathological conditions, such as, for example, RNA of the genome of the human immunodeficiency virus. In addition to the detection of RNAs associated with such viruses, it is desirable to detect other RNAs characteristic of, for example, a disease or pathological condition, such as those transcribed from a defective gene, as in the case of hemophilia or sickle-cell anemia. Characteristically, the RNA associated with such a disease or condition is present, if at all, in very small amounts relative to total nucleic acid in a given biological sample, such as a sample of blood or other body fluid or a tissue sample of an individual to be tested.

The detection of such a RNA species requires such specificity and sensitivity that, if the RNA is present, it is detectable and measurable from among the wide variety of other nucleic acid species with which it is associated in a sample. Some of these non-target nucleic acid species may bear close homology, at least in isolated segments, to the target RNA. Further, as noted above, these target RNA species are often found only in very minute amounts in a biological sample being tested. Yet, for proper diagnosis of the underlying disease state, it is essential that even such small amounts of a target RNA be detectable if present.

Several approaches have been advanced for accomplishing reliable detection, in a biological sample, of a target nucleic acid present, if at all, in only a small amount and as only a minute fraction of total nucleic acid. In one approach, the amount of nucleic acid in the sample is not altered. Instead, a reporter system is employed whereby a large number of readily detectable molecules is produced for each molecule of target nucleic acid in the sample and the presence or quantity of the detectable molecules is measured. Such a reporter system is a signal-generating system associated with the target nucleic acid and produces a detectable signal representative of the number of molecules of target nucleic acid in a sample.

In another, fundamentally different approach, a target nucleic acid segment, that is part of the target nucleic acid but not other nucleic acids in biological samples to be assayed, or the complement of such a target segment (i.e., the segment with the same size as, but sequence complementary to, that of the target segment), or both the target segment and its complement are selectively increased in copy number. That is, in a sample, the copy number of the target nucleic acid segment or its complement, or the copy numbers of both the target segment and its complement, are increased to a greater extent than the copy number of any other nucleic acid segment. This selective increase in copy number of a nucleic acid segment is referred to in the art as "amplification" of the segment. Once a target segment (also referred to herein, and in the art, as a "target sequence") or the complement of a target segment is amplified to a sufficient extent, it can be detected reliably by any of many techniques that have been developed in the nucleic acid probe art for the detection of nucleic acid segments, including techniques which involve the first approach, described above, that entails production of many readily detectable molecules for each molecule of target nucleic acid.

One method that has been developed for the amplification of a target segment is the so-called "polymerase chain reaction" ("PCR") method. This technique was reported by Saiki et al., Science 230, 1350 (1985) and Mullis et al., European Patent Application Publication Nos. 200362 and 201184 (See also U.S. Patent Nos. 4683195 and 4683202), and particularly entails repeated cycles of (1) hybridizing to the 3'-end of a target nucleic acid sequence a first primer and to the 3'-end of the sequence complementary to the target sequence a second primer, (2) extending the primers with a polymerase, and (3) rendering single stranded the duplexes resulting from the chain extension reactions. This PCR procedure results in amplification of the target sequence and its complement exponentially with the number of cycles (i.e., as in a chain reaction).

Certain RNAs are known to be susceptible to autocatalytic replication by certain polymerases, such as bacterial phage RNA-dependent RNA polymerases such as Qβ replicase. These RNAs are said herein to have "template sequences" which means they have sequences that make them templates for replication by the polymerase. In the process, the RNA made from a template RNA (i.e., an RNA with a "template sequence") in the reaction catalyzed by the replicase is also a replicatable RNA (i.e., also has a "template sequence"). Thus, in autocatalytic replication, the amount of replicatable RNA can increase exponentially. See Miele et al., J. Molecular Biology 171, 281 (1983).

Until recently it has not been appreciated that autocatalytic replication could be employed to provide convenient, broadly applicable, highly sensitive reporter systems for analyses of biological samples for the presence of particular nucleic acid sequences. A system in which probe for a target sequence is linked to an RNA capable of being replicated by Qβ replicase is described in PCT International Application Publication No. WO 87/06270 and by Chu et al., Nucleic Acids Research 14, 5591 (1986). Thus, the invention described in the PCT International Application Publication combines the art of replication of RNA with the art of oligonucleotide hybridization probes by providing for detection of target nucleic acid by replicative amplification of replicatable RNA associated (through a probe) with a target segment. WO 88/10315, which is a document according to Article 54(3)EPC, discloses co-functioning DNA-sequences each comprising a promoter, a replication-recognition site and a probe sequence and the use of a DNA-dependent RNA-polymerase for forming a replicatable RNA.

It is an object of the present invention as a selective embodiment to take further advantage of the basic RNA-replicative process for amplification, for ease in the detection of target RNA sequences, thus achieving exponential copying without the requirement necessarily of temperature cycling. It is a further object of the present invention to take advantage of RNA detection using a process that, in assuring the retention of the target sequence in the amplified product, avoids or at least substantially reduces the presence of false positives because the target is only detected after amplification if it was present in the sample probed. It is a further object of the present invention to combine the advantages of the replicative and extension product procedures as a means for detecting and measuring corresponding target RNA.

It is a basic object of the present invention to employ a first nucleotide sequence bearing a probe sequence and a sequence that is one operatively recognizable by a RNA-dependent RNA polymerase (replicase) for RNA-template-directed, primer initiated RNA strand synthesis (as well as initiation of autocatalytic replication) in conjunction with a second associated nucleotide sequence bearing a second probe sequence and a sequence that is also one operatively recognizable by said RNA-dependent RNA polymerase for RNA-template-directed, primer initiated RNA strand synthesis (as well as initiation of autocatalytic replication), such that an extension product of said first sequence serves as a template for preparation of an extension product of said second sequence, the latter extension product, either as such or in strand disassociated form being replicatable upon appropriate influence of the RNA-dependent RNA polymerase. Thus, the present invention relates to an amplification/detection system that reports only when RNA target sequence is present in the tested sample and whose amplification component operates exponentially without necessity of temperature cycling. It is thus a preferred object of the present invention to produce, by the initial event of hybridization to an intended, present target RNA sequence, a given extension product that corresponds by presence to target nucleic acid sequence which in turn leads to a subsequent product that is susceptible to amplification by replication to a plurality of RNA segments that in turn can be detected and measured such as via hybridization with an authentic target sequence and/or optionally by association with a signal grouping (i.e., a reporter group that is itself detectable or is capable in a suitable chemical reaction of leading to detectable molecules) that is accountable for their detection and measurement.

It is thus an overall object of the present invention to meet the goals enumerated by the art and to provide selective means to meet disadvantages and problems encountered by prior researchers' endeavors. The present invention utilizes reporter molecules that are present after amplification by replication, only when the necessary target nucleic acid sequence is present in the sample tested. It employs nucleic acid sequences that need only contain a sequence that is susceptible to hybridization potential, chain extension catalyzed by a replicase and ultimately to amplification by autocatalytic replication, and nothing more. Thus, the present invention provides means for detecting target nucleic acid sequences that are responsive only to presence of target RNA sequence itself. It further provides a straightforward technique that can be utilized reproducibly in an acceptably short period of time, employing the convenience of known reagents and having the precision necessary to reach consistent scientific results; one that can be employed in a reproducible assay setting and that is adaptable for use in kits for laboratory/clinical analyses. It is, hence, an object of the present invention to increase the detectability of certain RNA sequences (target segments) by amplification of sequences associated with the presence of the target sequences in an in vitro or exvivo system, utilizing in its preferred embodiments the advantages provided by the natural chain extension and replicative processes per se of replicases, and having the unique feature of being measurable only when target nucleic acid sequence is present.

### Summary of the Invention

Subject-matter of the present invention are cofunctioning nucleic acid primers according to claim 1 that are useful for forming an autocatalytically replicatable RNA which has incorporated therein a target RNA sequence. Preferred embodiments thereof are specified in claims 2 to 4.

Additional objects of the present invention are extension products according to claims 5 and 6 which are obtainable by employing the cofunctioning nucleic acid primers of claim 1.

Still another object of the invention is to provide a method for the detection of specific RNA target sequences in a nucleic acid containing sample according to claim 7. Preferred embodiments thereof are subject-matter of claims 8 to 20.

Still a further object of the invention is a kit according to claim 21 which is useful for the detection of a least one specific RNA target sequence in a nucleic acid containing sample.

The present invention is predicated on the use in a novel manner of a first nucleotide sequence comprising at its 5'-end, a first sequence, the complement of which sequence is a segment of a replicatable RNA which comprises the 3'-end of a replicatable RNA which is capable of RNA-dependent RNA polymerase directed autocatalytic replication and at its 3'-end, a first probe sequence which is capable of hybridizing to a first segment of a target RNA sequence, and priming an extension reaction, using the target RNA sequence as template, to produce a first primer extension product which includes an RNA sequence complementary to the target RNA sequence.

This first sequence operates herein in conjunction with a second nucleotide sequence, said second nucleotide sequence comprising
at its 5'-end, a second sequence the complement of which is capable of RNA-dependent RNA polymerase-directed catalytic replication and
at its 3'-end, a second probe sequence which is capable of hybridizing to a second segment of said complementary target sequence of said first primer extension product, wherein said second segment is located opposite to said first segment in said target sequence, and is capable of priming an extension reaction, using the first extension product as template, to produce a second primer extension product.

After hybridization of the first sequence to target sequence, if any, present in a sample being assayed for the target sequence, the first sequence is extended in a chain extension reaction catalyzed by the replicase. Then the extension product is separated from the target nucleic acid, which comprises the target sequence, and the second sequence is hybridized to the strand-separated extension product and extended in a second chain extension reaction by the replicase, provided that the RNA which consists of the second sequence joined at its 3'-end through a single phosphate to the 5'-end of the complement of the first sequence is autocatalytically replicatable by the replicase and provided further that an RNA which is made from said autocatalytically replicable RNA by inserting therein, between the 3'-end of said second replication-initiation sequence and the 5'-end of the complement of said first replication initiation sequence, is an RNA segment that is also autocatalytically replicatable by the replicase.

The product of the second extension, as is or in strand-separated form if necessary, is then, in a process hereof of amplification, autocatalytically replicated in the presence of the replicase. The amplified replication products are then detected and measured using any of numerous techniques known per se by the art skilled (see, e.g., PCT International Application Publication No. WO 87/06270).

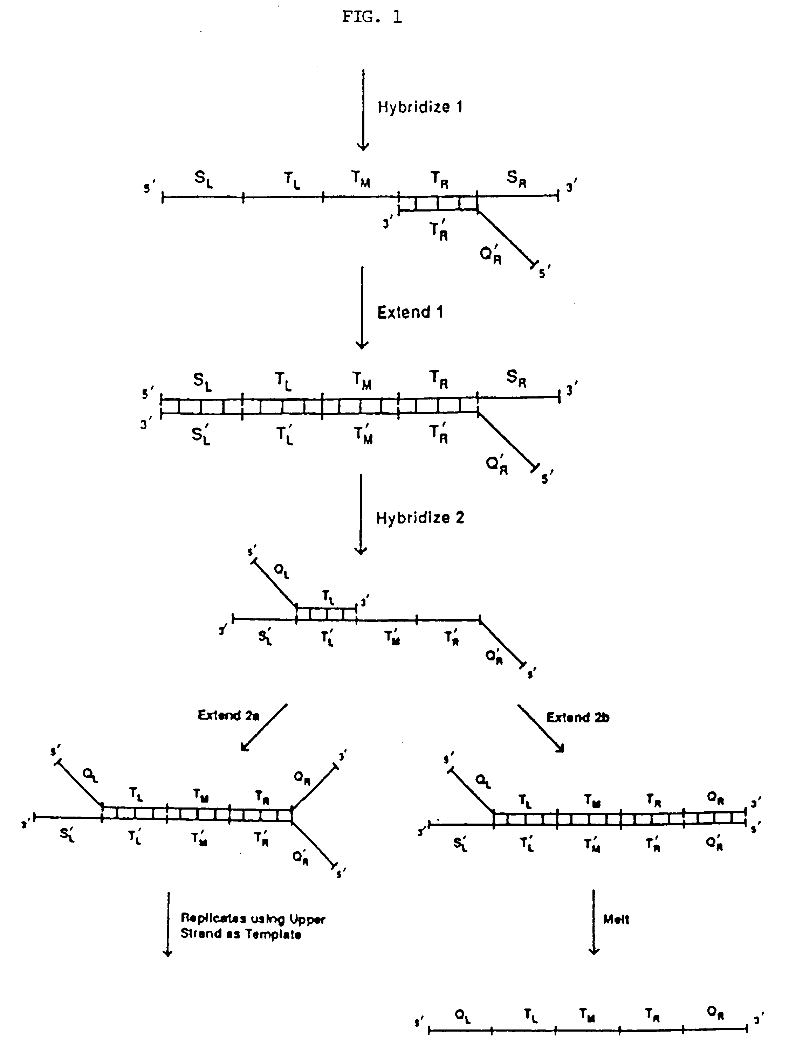

See Figure 1 hereof for a representative illustration of carrying out the target-sequence-recognition/amplification process aspect of the invention.

In an embodiment, the present invention is directed to novel, cofunctioning nucleotide sequences, their preparation and use, namely the first and second nucleotide sequences just described and described further below.

In another embodiment, the present invention is directed to an extension product.of said first nucleotide sequence, made after hybridization of said first nucleotide sequence with said target hybridized to said second nucleotide sequence
as well as an extension product of said second nucleotide sequence, made after hybridization of said second nucleotide sequence with the one strand of a strand-separated, first extension product, to which said second nucleotide sequence is capable of hybridizing through the second probe sequence.

The herein mentioned extension products are made in chain extension reactions that are catalyzed by an appropriate RNA-dependent RNA polymerase and carried out in the presence of the NTPs. In a preferred embodiment, said extension reaction(s) as well as the autocatalytic replication process is (are) conducted with the same replicase, namely, Q-beta replicase, where a sequence recognized by this enzyme is part of the extension product of the second nucleotide sequence hybridized to an extension product of the first nucleotide sequence (hybridized to, e.g., target sequence).

The products of the autocatalytic replication, which will contain replicase-recognizable sequence, are detected and measured in a manner known per se such as, for example, via a chromophore moiety or a radioactive moiety incorporated in the course of the autocatalytic replication or by hybridization with an oligonucleotide probe which is detectably labeled and comprises at least a subsequence of the target sequence or the complement of such a subsequence.

In all respects, the present invention is directed to the novel application of the natural principles of hybridization of complementary RNA sequences, chain extension of RNA primers on RNA templates catalyzed by replicases (see, e.g., Vournakis et al., Biochem. Biophys. Res. Commun. 70. 774 (1976)), and autocatalytic replication of many RNAs, including recombinant RNAs, by replicases (see, e.g., Miele et al., supra; United States Patent No. 4,786,600 ) in order to amplify and render detectable and measureable target RNA sequences of target RNAs that may be present in a biological sample containing a mixture of nucleic acids.

The present invention is further directed to methods and means for assay systems based upon such principles and to kits incorporating components necessary for such assay methodology for detecting or measuring target RNA sequences, including in laboratory and clinical settings.

The present invention thus provides a method for the detection of at least one specific RNA target, in a sample containing nucleic acid, said RNA target comprising a target sequence and said method comprising detecting replicatable extension product (or complement thereof), said product being the product of extension from a second ribonucleotide (i.e., RNA) sequence hybridized with a first RNA extension product strand-separated from the target RNA, which provides the template for synthesis of the first extension product from a first ribonucleotide sequence hybridized with the 3'-end of the target sequence. In the method, said first ribonucleotide sequence comprises a first probe sequence complementary to a subsequence at the 3'-end of the target sequence and, 5' from and contiguous with (i.e., linked through a single phosphate to) the 5'-terminal ribonucleotide of the probe sequence, a first sequence that (a) is one capable of being recognized by a replicase for initiation of an RNA synthesis (i.e., chain extension) process from the 3'-end of the probe sequence utilizing target RNA sequence as a template and (b) is a part, including the 5'-end, of an RNA that is autocatalytically replicatable by the replicase. Further, said second ribonucleotide sequence comprises (1) a second probe sequence that has the same sequence as a subsequence at the 5'-end of the target sequence (said 5'-subsequence not overlapping the 3'-subsequence of which the first probe sequence is the complement) and, therefore, a sequence that is complementary to that of a subsequence of the extension product from the first nucleotide sequence hybridized to target RNA, the 5'-end of said subsequence being 3' from the 3'-end of the first nucleotide sequence in said extension product; and (2) 5'-from and contiguous with said second probe sequence, a second sequence that (a) is one capable of being recognized by the replicase for initiation of an RNA synthesis (i.e., chain extension) process from the 3'-end of the second probe sequence utilizing complement of target RNA sequence as a template and (b) is a part, including the 5'-end, of an RNA that is autocatalytically replicatable by the replicase, provided that the RNA which consists of the second sequence joined at its 3'-end through a single phosphate to the 5'-end of the complement of the first sequence is autocatalytically replicable by the replicase and provided further that an RNA which is made from said autocatalytically replicable RNA by inserting therein, between the 3'-end of said second sequence and the 5'-end of the complement of said first sequence, an RNA segment that is also autocatalytically replicable by the replicase. The replicatable extension product of said second nucleotide sequence functions, by autocatalytic replication catalyzed by the replicase, to amplify the target sequence. The replicatable extension product of the second nucleotide sequence as well as the complement of said extension product, by virtue of being autocatalytically replicated, also serve as reporter molecules for the target sequence and associated target RNA.

The present invention advantageously combines the use of replication (i.e., autocatalytic replication), thus providing selective, exponential growth of the replicated nucleic acids without temperature cycling required in processes such as PCR, with inclusion of the target RNA sequence in the replicatable product, thereby providing amplification of the target sequence, rather than simply a number of reporter molecules for each molecule of target. The present invention also advantageously utilizes the capability of RNA-dependent RNA-polymerases, such as Q-beta replicase, to both synthesize RNAs from a primer using an RNA as a template and autocatalytically replicate RNAs, thus providing for amplification of target and production of reporter molecules with a single enzyme.

The present invention further embodies means for detecting and measuring the amount of the products of the autocatalytic replication and, thereby, also measuring the amount of target RNA present in a sample being analyzed.

In an aspect, the present invention is directed to a method useful for the detection of at least one specific RNA target sequence in a sample containing nucleic acid, comprising:
hybridizing with said target RNA sequence under suitable conditions a first nucleotide sequence comprising a first probe sequence which is complementary in sequence to a segment of said target sequence and its 5'-end a funtional length of a first sequence that is the complement of one susceptible to 5 replication upon association with an appropriate RNA-dependent RNA polymerase,
chain extending said hybridized nucleotide sequence,
strand separating the extension product,
hybridizing with the strand separated in the previous step and containing the sequence that is the complement of one susceptible to replication a second nucleotide sequence comprising at its 3'-end a second probe sequence capable of hybridizing with said separated strand at the end opposite of the sequence that is the complement of said target sequence and at its 5'-end a functional length of a second sequence that is the complement of one susceptible to replication upon association with an appropriate RNA-dependent RNA polymerase,
chain extending said hybridized second nucleotide sequence,
permitting operatively the second extension product of the previous step, optionally after strand separation, to undergo replication by contact with an appropriate RNA-dependent RNA polymerase, and
detecting the replication products.

The present invention, in application, embodies the detection of said amplification products such as via radio- or chromophore-labeling or hybridization techniques known per se.

The present invention contemplates the detection of RNA target sequence in a sample wherein said target sequence is one associated with characteristics of a genetic or pathogenic disease or condition, and particularly those wherein the target RNA sequence is a segment of RNA of a human virus or a transcript of a defective gene or a defective transcript of a normal gene.

There are a number of human diseases that are either the direct result of a genetic defect or are correlated with the presence of a particular genetic allele. By way of example, the technique described in this application could be used to determine whether or not a given target is present in a very small sample of nucleic acid. This would be useful in the diagnosis of genetic disorders via the detection of corresponding mRNA species or in the testing for presence of viral infection, e.g., HIV-1.

The present invention contemplates the use of appropriate RNA-dependent RNA polymerase (replicase) enzymes that are capable both of chain extension and replication. A preferred embodiment employs Q-beta replicase enzyme to achieve both functions in a convenient, so-called single-pot reaction.

The present invention is also directed to assay systems and kits embodying same, useful for the detection of at least one specific RNA target sequence in a sample containing nucleic acid, after amplification of the target RNA sequence in accordance with the present invention. Kits would comprise a replicase, a first nucleotide sequence for carrying out the amplification process, as described above, a second nucleotide sequence for carrying out the amplification process, as described above, and compositions required to detect or provide detectability to the amplified product.

### Detailed Description of the Invention

### 1. Brief description of the drawing

Figure 1 depicts schematically an aspect of this invention, namely the steps hereof in target amplification using a single- or double-stranded RNA template for replication. Extension and amplification are carried out, e.g., with Q-beta RNA polymerase (replicase). T(target) is redesignated in segmented fashion as T_{L}T_{M}T_{R}. The sequences Q_{L}T_{L} and T_{R}'Q_{R}' may be obtained by molecular cloning and subsequent in vitro transcription. The required template for autocatalytic replication amplification is obtained by hybridization, extension, rehybridization and reextension as shown. Where the Q sequences represent Q-beta sequences for initiation of chain extension and replication, Q-beta replicase both chain extends and replicates, in turn.

### 2. General methods and definitions

Reference is made to standard textbooks of molecular biology that contain definitions and methods and means for carrying out basic techniques of the present invention, such as:
RNA probe or primer preparation, including transcription of encoding DNA in an expression vector and the tailoring thereof so as to be suitable as such or when linked to other RNA for use as a probe herein;
preparation of nucleotides with different functional sequences for use in hybridization;
hybridization methodology including variations in stringency conditions for producing more or less hybridization certainty depending on the degree of homology of the primer to a target RNA sequence;
identification, isolation or preparation of RNA polymerases capable of chain extension reactions and of recognizing said replicatable sequences referred to above;
conditions conducive to the initiation and maintenance of extension reactions including use of RNA-dependent RNA polymerase and NTPs;
the mechanism and methodology for autocatalytic (sometimes referred to herein as "induced") replication; and so forth.

See, for example, Maniatis et al., Molecular Cloning: A Laboratory Manual, Cold Spring Harbor Laboratory, New York 1982), and Colowick et al., Methods in Enzymology Volume 152, Academic Press, Inc. (1987), and the various references cited therein.

All of the aforecited publications are by this reference hereby expressly incorporated herein.

By the term "probe" in the present context is meant a RNA sequence that has sufficient homology with the target sequence such that under suitable hybridization conditions it is capable of hybridizing, that is binding to, the target sequence. A typical probe is at least about 10 nucleotides in length, and most preferably is of approximately 25 or more nucleotide bases in length, and in its most preferred embodiments, it shares identity or very high homology with the target sequence. See, for example, EPT-A-128042 (publd. 12 Dec 84).

The techniques of forming a detection signal such as via radioactive labeling or chromogenic means using a chromogenic susceptible enzyme are also well known and documented in the art.

A sample on which the assay method of the invention is carried out can be a raw specimen of biological material, such as serum or other body fluid, tissue culture medium or food material. More typically, the method is carried out on a sample which is a processed specimen, derived from a raw specimen by various treatments to remove materials that would interfere with detection of target, such as by causing non-specific binding of affinity molecules. Methods of processing raw samples to obtain a sample more suitable for the assay methods of the invention are well known in the art.

Reference herein to bacteriophage Qβ is not limited to any particular variant or mutant or population thereof. Such reference, unless otherwise specifically limited, is to any variant, mutant or population which, upon infection therewith of E. coli susceptible to bacteriophage Qβ infection, is capable of causing production of an RNA-dependent RNA-polymerase or any polymerase acting as a replicase and its associated nucleic acid substrate.

For other phages which, upon infection of bacteria susceptible to infection therewith, produce RNA-dependent RNA polymerases, and associated replicatable RNAs capable of being autocatalytically replicated in vitro, which can be employed in the present invention, see, e.g., Miyake et al., Proc. Natl. Acad. Sci. (U.S.A.) 68, 2022 (1971).

RNA resulting from the replication process can be made fluorescent by employing a T4 RNA ligase catalyzed reaction to append nucleotides modified to be fluorescent to the 3'-end of replicative RNA. See Cosstick et al., Nucl. Acids Res. 12, 1791 (1984). The fluorescence of the resulting RNA can be employed to detect the RNA by any of several standard techniques.

Among still other methods that can be used to detect replicated RNA are those wherein a reporter substance, that binds specifically with nucleic acid, is added to the system in which the replication has taken place, or to the medium, such as a positively charged support such as ECTEOLA paper, on which replicated RNA has been isolated, and signal from the reporter substance measured. Such substances include: chromogenic dyes, such as "stains all" (Dahlberg et al., J. Mol. Biol. 41, 139 (1969); methylene blue (Dingman et al., Biochemistry 7, 659 (1968), and silver stain (Sammons et al., Electrophoresis 2 , 135 (1981); Igloi, Anal. Biochem. 134, 184 (1983)); fluorogenic compounds that bind to RNA -- for example, ethidium bromide (Sharp et al., Biochemistry 12, 3055 (1973); Bailey et al., Anal. Biochem. 70, 75 (1976); and fluorogenic compounds that bind specifically to RNAs that are templates for replication by Qβ replicase -- for example, a phycobiliprotein (Oi et al., J. Cell Biol. 93, 981 (1982); Stryer et al., U.S. Patent No. 4,520,110) conjugated to the viral subunit of Qβ replicase.

Provided that the concentration of replicase remains above the concentration of template RNA, and that ribonucleoside-5'-triphosphate concentration does not become limiting, the concentration of template RNA will increase exponentially with time during replicase-catalyzed RNA replication. After template RNA concentration equals or exceeds replicase concentration, as long as ribonucleoside-5'-triphosphate concentration does not become limiting, the concentration of template RNA will increase linearly with time. See, e.g., Kramer et al. (1974), supra.

It has been found that, under the conditions for replicase-catalyzed replication, the MDV-1 RNA there exemplified doubled in concentration every 36 seconds, until template concentration exceeded enzyme concentration.

The concentration of template RNA, in a replicase-catalyzed replication reaction system after a given time for reaction, will be related to the initial concentration of template RNA. If, at all times during the replication reaction, the concentration of replicase exceeds that of template (and ribonucleoside-5'-triphosphate concentration does not become limiting), the log of concentration of template RNA at the conclusion of the reaction will be directly proportional to the log of the initial concentration of template (at the start of the reaction). After replicase concentration falls below template concentration, as long as ribonucleoside-5'-triphosphate concentration does not become limiting, the concentration of template at the conclusion of reaction is directly proportional to the log of the initial concentration of template. Further, the time required for a reaction to reach the point at which template concentration equals replicase concentration is proportional to the negative log of the initial concentration of template.

By allowing the replication reaction to proceed for longer times, greater sensitivity can be achieved.

In assays according to the invention, assays are carried out simultaneously, under conditions as nearly alike as possible, on both test samples, which are being tested for target, and control samples. As understood in the art, control samples are similar to test samples but are known to contain either no target or a known quantity of target. A control with no target establishes the "background," below which it is not possible to distinguish samples which contain target from those which do not. By comparing the amount or concentration of replicated replicative RNA produced in an assay of a test sample with the amount or concentration produced with control samples assayed simultaneously, the presence of target in test sample at a level above background can be determined. If control samples with a range of known concentrations of target are employed, the concentration of target in a test sample can be estimated.

Again, the use of a "replicase" for (autocatalytic) induction of replication of the RNA products of the present invention are generally known in the art. Suitable examples of such replicases that are useful in the present invention include the so-called Qβ virus replicase that recognizes certain nucleic acid sequence sites at the 3'-end of the given RNA transcript. These replicases serve to replicate, that is reproduce, the RNA transcripts and complements so as to multiply copies thereof. When such enzyme is present in the reaction locus during the process of transcription, it can be foreseen that the multiple transcripts that are produced during transcription can themselves undergo replication so as to exponentially increase the amount of RNA transcript product.

The following examples illustrate a model system of this invention:

### 4. Examples

Exemplified is the use of Q-beta polymerase and an RNA substrate that is replicatable by said polymerase in order to amplify a target RNA sequence that is contained within the RNA substrate. Q-beta replicase is an RNA-dependent RNA polymerase that recognizes characteristic structural elements at the 3' end of an RNA substrate and subsequently produces a complementary copy of the substrate. If the complementary copy also has the requisite structural elements at its 3' end, then it too can be recombinized and copied by Q-beta replicase, resulting in an autocatalytic reaction cycle that exponentially amplifies the substrate sequence
Q-beta replicase is able to bypass its normal initiation specificity and extend complementary synthesis from the 3' end of a suitable oligonucleotide primer (Felix, G. & Hake, H. Biochem. Biophys. Res. Comm. 65, 503-509, 1975). The reaction generates mainly partial-length cRNAs and a considerable amount of non-specific RNAs (Vournakis et al., Biochem. Biophys. Res. comm. 70, 774-782, 1976), and thus is not suitable for replication. However, it could be used to extend an RNA primer through a short target region of about 20-100 nucleotides.

### Preparation of Primers

Two RNA primers are prepared by in vitro transcription of a suitably constructed recombinant DNA. The first primer contains the first 157 nucleotides (at the 5'-end) of the minus-strand of Q-beta MDV-1 RNA followed by 10 - 50 nucleotides that are complementary to the target RNA over a region extending 5' from the 3' end (i.e. "just downstream from") of the site of interest (i.e., the target sequence). The second primer contains the first 61 nucleotides (at the 5' end) of the plus-strand of Q-beta MDV-1 RNA followed by 10-50 nucleotides that are identical to the target RNA over a region extending 3' from the 5'-end of the target sequence.

### Hybridization and Primer Extension

A control template DNA, such as pT7-0 (U.S. Biochemical), is used to prepare suitable RNA transcripts that can serve as a target for detection. 1 fg, 10 fg, 100 fg, 1 pg, 10 pg, or 100 pg of the RNA transcript (10⁻⁵ fmol - 10³ pmol) is diluted to 50 µl volume to give a final solution containing 100 mM Tris·Hcl (pH 7.5), 22 mM Mgcl₂, 2 mM Na₂EDTA, and 1 mM (each) of the four NTPs. To this solution is added a 25 µl volume containing 2 ng (approx. 1 nM) of each of the two RNA primers. The mixture is heated to 70°C for 1 min and then quick-cooled on ice. A 25 µl volume containing 2 µg of Q-beta replicase is added, and the mixture is incubated at 37°C for 10 min. The mixture is again heated to 70°C for 1 min and quick-cooled on ice. A second 25 µl volume containing 2 µg of Q-beta replicase is added, and the mixture is again incubated at 37°C for 10 min.

### Amplification of Taraet RNA

The second primer-extension product is optionally released from the template by heating to 70°C for 1 min and quick-cooling on ice. If this step is included, then a third 25 µl volume containing 2 µg of Q-beta replicase in a solution containing 50 mM Tris·Hcl (pH 7.5), 11 mM Mgcl₂,1 mM Na₂EDTA, and 0.5 mM (each) of the four NTPs must be added. In either case, amplification of the target RNA then proceeds autocatalytically by incubating at 37°C for 20 min. The resulting mixture can then be assayed for the production of MDV-1 RNA which contains an insert that corresponds to the desired target sequence.

### Detection of Replicated RNA

The amount of RNA is determined by its intrinsic UV absorbance (e.g. as by the contact photoprinting method of Kutateladze et al., Anal. Biochem. 100, 129 (1979)). Alternatively, the RNA is visualized on ETEOLA paper. Aliquots (of equal volume) of replication reaction are transferred with 13, 48 or 96-fingered aliquotter to sheets of diethylaminoethyl cellulose paper. The sheets are then washed at room temperature in a solution of 200 mM NaCl, 300 mM ammonium acetate pH 6 to remove ribonucleoside triphosphates not incorporated into RNA. The sheets are then stained with 0.3 µg/ml of ethidium bromide. (Sharp et al., Biochemistry 12, 3055 (1973); Bailey et al., Anal. Biochem 70, 75 (1976).

Finally the fluorescence from individual blots is measured by any of several known techniques. Fluorescence intensity from a stained blot above that from control blots indicates the presence of target. Other staining materials can be employed in place of ethidium bromide. These include methylene blue (Dingman and Peacock, Biochemistry 7, 659 (1968)), silver stain (Sammons, et al., Electrophoresis 2, 135 (1981)) or phycobiliprotein Qβ replicase conjugate (Oi et al., J. Cell Biol. 93, 981 (1982)).

The foregoing description details more specific methods that can be employed to practice the present invention and represents the best mode contemplated. However detailed the foregoing may appear in text, it should not be construed as limiting the overall scope hereof.

## Claims

1. Co-functioning nucleic acid primers useful for forming an autocatalytically replicatable RNA which has incorporated therein a target RNA sequence, said co-functioning primers comprising:
(1) a first nucleic acid primer comprising:
(a) at its 5'-end, a first sequence, the complement of which sequence is a segment of a replicatable RNA which comprises the 3'-end of a replicatable RNA which is capable of RNA-dependent RNA polymerase directed autocatalytic replication and
(b) at its 3'-end, a first probe sequence which is capable of hybridizing to a first segment of a target RNA sequence, and priming an extension reaction, using the target RNA sequence as template, to produce a first primer extension product which includes an RNA sequence complementary to the target RNA sequence; and
(2) a second nucleic acid primer comprising:
(a) at its 5'-end, a second sequence the complement of which is capable of RNA-dependent RNA polymerase-directed catalytic replication and
(b) at its 3'-end, a second probe sequence which is capable of hybridizing to a second segment of said complementary target sequence of said first primer extension product, wherein said second segment is located opposite to said first segment in said target sequence, and priming an extension reaction, using the first extension product as template, to produce a second primer extension product which is said target sequence-containing autocatalytically replicatable RNA; provided that the RNA which consists of the second sequence joined at its 3'-end through a single phosphate to the 5'-end of the complement of the first sequence is autocatalytically replicatable by the replicase and provided further that an RNA which is made from said autocatalytically replicable RNA by inserting therein, between the 3'-end of said second replication-initiation sequence and the 5'-end of the complement of said first replication initiation sequence, is an RNA segment that is also autocatalytically replicatable by the replicase.

2. Nucleic acid primers according to Claim 1 wherein said RNA-dependent RNA polymerase is Q-beta replicase.

3. Nucleic acid primers according to Claim 1 or 2 wherein said target sequence corresponds to an RNA segment of a human immunodeficiency virus.

4. Nucleic acid primers according to Claim 1 or 2 wherein said target sequence is a transcript of a defective gene or a defective transcript of a normal gene.

5. A strand-separated extension product of the first nucleic acid primer according to claim 1 hybridized to a second nucleic acid primer according to claim 1.

6. An extension product of the hybridization complex according to claim 5, wherein said extension product, in the presence of replicase, autocatalytically replicates the target sequence.

7. A method for the detection of at least one specific RNA target sequence in a nucleic acid containing sample, said method comprising detecting replicatable extension product, said product being the product of extension from a second nucleic acid primer hybridized with a strand separated from a first extension product that contains a sequence of a first nucleic acid primer hybridizable with a target RNA sequence, said replicatable extension product functioning as a reporter molecule for said target,
wherein said first nucleic acid primer comprises:
(a) at its 5'-end, a first sequence, the complement of which sequence is a segment of a replicatable RNA which comprises the 3'-end of a replicatable RNA which is capable of RNA-dependent RNA polymerase-directed autocatalytic replication and
(b) at its 3'-end, first probe sequence which is capable of hybridizing to a first segment of a target RNA sequence, and priming an extension reaction, using the target RNA sequence as template, to produce a first primer extension product which includes an RNA sequence complementary to the target RNA sequence;
and wherein said second nucleic acid primer comprises:
(a) at its 5'-end, a second sequence the complement of which is capable of RNA-dependent RNA polymerase-directed autocatalytic replication and
(b) at its 3'-end, a second probe sequence which is capable of hybridizing to a second segment of said complementary target sequence of said first primer extension product, wherein said second segment is located opposite to said first segment in said target sequence, and priming an extension reaction, using the first primer extension product as template, to produce a second primer extension product which is said target sequence-containing autocatalytically replicatable RNA; provided that the RNA which consists of the second sequence joined at its 3'-end through a single phosphate to the 5'-end of the complement of said first replication initiation sequence, is an RNA segment that is also autocatalytically replicatable by the replicase.

8. A method according to Claim 7 including the additional step of detecting replicatable product after permitting said product to replicate.

9. A method according to Claim 8 wherein said replication is effected by contacting replicatable product with replicase enzyme.

10. A method according to Claim 9 wherein said replicase enzyme is Q-beta replicase.

11. A method according to claim 7 comprising:
hybridizing with said target RNA sequence under suitable conditions a first nucleic acid primer, said first nucleic acid primer comprising at its 3'-end, a first probe sequence which is complementary to a first segment of said target sequence and, at its 5'-end, a first sequence, the complement of which comprises a replicatable RNA capable of replication upon association with an appropriate RNA-dependent RNA polymerase,
chain extending said hybridized first nucleic acid primer, strand separating the resulting extension product, hybridizing said separated strand with a second nucleic acid primer, said second nucleic acid primer comprising at its 3'-end, a second probe sequence which is capable of hybridizing to a second segment of said complementary target sequence of said separated strand, wherein said second segment is located opposite to said first segment in said target sequence, and at its 5'-end, a functional length of a second sequence, the complement thereof being susceptible to replication upon association with an appropriate RNA-dependent RNA polymerase,
chain extending said hybridized second nucleic acid primer, permitting the second extension product produced thereby, optionally after strand separation, to undergo replication by contacting said second extension product with an appropriate RNA-dependent RNA polymerase, and detecting the replication product.

12. The method according to Claim 11 wherein said RNA-dependent RNA polymerase is Q-beta replicase.

13. The method according to Claim 11 or 12 wherein the detected product is measured in a standardized manner so as to measure the amount of target sequence contained in a sample of nucleic acid.

14. The method according to Claim 11, 12 or 13 wherein said target sequence is contained within a nucleic acid sequence associated with a genetic or pathogenic disease or condition.

15. The method according to Claim 14 wherein said nucleic acid sequence corresponds to an RNA segment of a human immunodeficiency virus.

16. The method according to Claim 14 wherein said nucleic acid sequence is a transcript of a defective gene or a defective transcript of a normal gene.

17. The method according to Claim 11 or 12 wherein said detected products are labeled prior to detection.

18. The method according to Claim 17 wherein said products are radio-labeled.

19. The method according to Claim 17 wherein said products are chromophore labeled.

20. The method according to any one of Claims 11 to 16 wherein said detecting is conducted by hybridization of the replicated product with an authentic, optionally labeled, target sequence.

21. A kit useful for the detection of at least one specific RNA target sequence in a sample containing nucleic acid, said kit comprising:
(1) a first nucleic acid primer comprising:
(a) at its 5'-end, a first sequence, the complement of which sequence is a segment of a replicatable RNA which comprises the 3'-end of a replicatable RNA which is capable of RNA-dependent RNA polymerase directed autocatalytic replication and
(b) at its 3'-end, a first probe sequence which is capable of hybridizing to a first segment of a target RNA sequence, and priming an extension reaction, using the target RNA sequence as template, to produce a first primer extension product which includes an RNA sequence complementary to the target RNA sequence; and
(2) a second nucleic acid primer comprising:
(a) at its 5'-end, a second sequence the complement of which is capable of RNA-dependent RNA polymerase-directed catalytic replication and
(b) at its 3'-end, a second probe sequence which is capable of hybridizing to a second segment of said complementary target sequence of said first primer extension product, wherein said second segment is located opposite to said first segment in said target sequence, and priming an extension reaction, using the first extension product as template, to produce a second primer extension product which is said target sequence-containing autocatalytically replicatable RNA; provided that the RNA which consists of the second sequence joined at its 3'-end through a single phosphate to the 5'-end of the complement of the first sequence is autocatalytically replicatable by the replicase and provided further that an RNA which is made from said autocatalytically replicatable RNA by inserting therein, between the 3'-end of said second replication-initiation sequence and the 5'-end of the complement of said first replication initiation sequence, is an RNA segment that is also autocatalytically replicatable by the replicase and
(3) an RNA-dependent RNA polymerase.

## Patentansprüche

1. Kofunktionelle Nukleinsäureprimer, die zum Bilden einer autokatalytisch replizierbaren RNA, die eine Ziel-RNA-Sequenz enthält, geeignet sind, wobei die kofunktionellen Primer umfassen:
(1) einen ersten Nukleinsäureprimer, umfassend:
(a) an seinem 5'-Ende eine erste Sequenz, von welcher Sequenz das Komplement ein Segment einer replizierbaren RNA ist, umfassend das 3'-Ende einer replizierbaren RNA, die zur durch RNA-abhängige RNA-Polymerase erfolgenden aptokatalytischen Replikation fähig ist und
(b) an seinem 3'-Ende eine erste Sondensequenz, die fähig ist zum Hybridisieren an ein erstes Segment einer Ziel-RNA-Sequenz und zum Starten einer Extensionsreaktion unter Verwendung der Ziel-RNA-Sequenz als Matrize, um ein erstes Primerextensionsprodukt zu erzeugen, welches eine zu der Ziel-RNA-Sequenz komplementäre RNA-Sequenz umfaßt, und
(2) einen zweiten Nukleinsäureprimer, umfassend
(a) an seinem 5'-Ende eine zweite Sequenz, deren Komplement zu einer durch RNA-abhängige RNA-Polymerase erfolgenden katalytischen Replikation fähig ist und
(b) an seinem 3'-Ende eine zweite Sondensequenz, die fähig ist zum Hybridisieren an ein zweites Segment der komplementären Zielsequenz des ersten Primerextensionsprodukts, worin das zweite Segment gegenüberliegend zu dem ersten Segment in der Zielsequenz angeordnet ist und zum Starten einer Extensionsreaktion, unter Verwendung des ersten Extensionsprodukts als Matrize, um ein zweites Primerextensionsprodukt zu erzeugen, das die, die Zielsequenz enthaltende autokatalytisch replizierbare RNA ist, mit der Maßgabe, daß die RNA, die aus der zweiten Sequenz, die mit ihrem 3'-Ende über ein einzelnes Phosphat an das 5'-Ende des Komplements der ersten Sequenz gebunden ist, besteht, autokatalytisch durch die Replikase replizierbar ist und mit der weiteren Maßgabe, daß eine RNA, die aus der autokatalytisch replizierbaren RNA dadurch hergestellt ist, daß darin zwischen dem 3'-Ende der zweiten Replikationsinitiationssequenz und dem 5'-Ende des Komplements der ersten Replikationsinitiationssequenz ein RNA-Segment insertiert wird, das ebenfalls autokatalytisch durch die Replikase replizierbar ist.

2. Nukleinsäureprimer nach Anspruch 1, worin die RNA-abhängige RNA-Polymerase Q-beta-Replikase ist.

3. Nukleinsäureprimer nach Anspruch 1 oder 2, worin die Zielsequenz einem RNA-Segment eines menschlichen Immunschwächevirus entspricht.

4. Nukleinsäureprimer nach Anspruch 1 oder 2, worin die Zielsequenz ein Transkript eines defekten Gens oder eines defekten Transkripts eines normalen Gens ist.

5. Stranggetrenntes Extensionsprodukt eines ersten Nukleinsäureprimers gemäß Anspruch 1, das an einen zweiten Nukleinsäureprimer nach Anspruch 1 hybridisiert ist.

6. Extensionsprodukt des Hybridisierungskomplexes nach Anspruch 5, worin das Extensionsprodukt in der Gegenwart von Replikase autokatalytisch die Zielsequenz repliziert.

7. Verfahren zum Nachweis von wenigstens einer spezifischen RNA-Zielsequenz in einer Nukleinsäure enthaltenden Probe, wobei das Verfahren das Nachweisen eines replizierbaren Extensionsprodukts umfaßt, wobei das Produkt das Produkt einer Extension eines zweiten Nukleinsäureprimers, der mit einem Strang hybridisiert ist, der von einem ersten Extensionsprodukt getrennt ist, das eine Sequenz von einem ersten, mit einer Ziel-RNA-Sequenz hybridisierbaren Nukleinsäureprimer enthält, wobei das replizierbare Extensionsprodukt als ein Signalmolekül für das erste Ziel dient,
worin der erste Nukleinsäureprimer umfaßt:
(a) an seinem 5'-Ende eine erste Sequenz, von welcher Sequenz das Komplement ein Segment einer replizierbaren RNA ist, umfassend das 3'-Ende einer replizierbaren RNA, die zu einer durch RNA-abhängige RNA-Polymerase erfolgenden autokatalytischen Replikation fähig ist und
(b) an seinem 3'-Ende eine erste Sondensequenz, die fähig ist zum Hybridisieren an ein erstes Segment einer Ziel-RNA-Sequenz und zum Starten einer Extensionsreaktion unter Verwendung der Ziel-RNA-Sequenz als Matrize, um ein erstes Primerextensionsprodukt zu erzeugen, welches eine zu der Ziel-RNA-Sequenz komplementäre RNA-Sequenz umfaßt, und worin der zweite Nukleinsäureprimer umfaßt:
(a) an seinem 5'-Ende eine zweite Sequenz, deren Komplement zu einer durch RNA-abhängige RNA-Polymerase erfolgenden katalytischen Replikation fähig ist und
(b) an seinem 3'-Ende eine zweite Sondensequenz, die fähig ist zum Hybridisieren an ein zweites Segment der komplementären Zielsequenz des ersten Primerextensionsprodukts, worin das zweite Segment gegenüberliegend zu dem ersten Segment in der Zielsequenz angeordnet ist und zum Starten einer Extensionsreaktion, unter Verwendung des ersten Primerextensionsprodukts als Matrize, um ein zweites Primerextensionsprodukt zu erzeugen, das die, die Zielsequenz enthaltende autokatalytisch replizierbare RNA ist, mit der Maßgabe, daß die RNA, die aus der zweiten Sequenz, die mit ihrem 3'-Ende über ein einzelnes Phosphat an das 5'-Ende des Komplements der ersten Replikationsinitiationssequenz gebunden ist, besteht, ein RNA-Segment ist, das ebenfalls durch die Replikase autokatalytisch replizierbar ist.

8. Verfahren nach Anspruch 7, umfassend den zusätzlichen Schritt des Nachweisens von replizierbarem Produkt, nachdem man eine Replikation des Produkts zugelassen hat.

9. Verfahren nach Anspruch 8, worin die Replikation durch Kontaktieren von replizierbarem Produkt mit Replikaseenzym bewirkt wird.

10. Verfahren nach Anspruch 9, worin das Replikaseenzym Q-beta Replikase ist.

11. Verfahren nach Anspruch 7, umfassend:
Hybridisierung eines ersten Nukleinsäureprimers mit der Ziel-RNA-Sequenz unter geeigneten Bedingungen, wobei der erste Nukleinsäureprimer an seinem 3'-Ende eine erste Sondensequenz, die komplementär zu einem ersten Segment der Zielsequenz ist, und an seinem 5'-Ende eine erste Sequenz umfaßt, deren Komplement eine replizierbare RNA umfaßt, die zur Replikation durch Assoziation mit einer geeigneten RNA-abhängigen RNA-Polymerase fähig ist,
Kettenextension des hybridisierten ersten Nukleinsäureprimers, Strangtrennung des resultierenden Extensionsprodukts, Hybridisierung des abgetrennten Stranges mit einem zweiten Nukleinsäureprimer, wobei der zweite Nukleinsäureprimer an seinem 3'-Ende eine zweite Sondensequenz umfaßt, die zum hybridisieren an ein zweites Segment der komplementären Zielsequenz des getrennten Strangs fähig ist, worin das zweite Segment gegenüber dem ersten Segment in der Zielsequenz angeordnet ist, und an seinem 5'-Ende ein funktionelles Stück einer zweiten Sequenz, von welcher das Komplement durch Assoziation mit einer geeigneten RNA-Polymerase-abhängigen RNA-Polymerase einer Replikation zugänglich ist,
Kettenverlängerung des hybridisierten zweiten Nukleinsäureprimers, Zulassen, daß, gegebenenfalls nach der Strangtrennung, das dadurch erzeugte zweite Extensionsprodukt durch Kontaktieren des zweiten Extensionsprodukts mit einer geeigneten RNA-abhängigen RNA-Polymerase eine Replikation eingeht und Nachweis des Replikationsprodukts.

12. Verfahren nach Anspruch 11, worin die RNA-abhängige RNA-Polymerase Q-beta-Replikase ist.

13. Verfahren nach Anspruch 11 oder 12, worin das nachgewiesene Produkt auf eine standardisierte Art gemessen wird, um die Menge der in einer Nukleinsäureprobe enthaltene Menge an Zielsequenz zu messen.

14. Verfahren nach Anspruch 11, 12 oder 13, worin die Zielsequenz in einer Nukleinsäuresequenz enthalten ist, die mit einer genetischen oder pathogenen Erkrankung zusammenhängt.

15. Verfahren nach Anspruch 14, worin die Nukleinsäuresequenz einem RNA-Segment eines menschlichen Immunschwächevirus entspricht.

16. Verfahren nach Anspruch 14, worin die Nukleinsäuresequenz ein Transkript eines defekten Gens oder ein defektes Transkript eines normalen Gens ist.

17. Verfahren nach Anspruch 11 oder 12, worin die nachgewiesenen Produkte vor dem Nachweisen markiert werden.

18. Verfahren nach Anspruch 17, worin die Produkte radioaktiv markiert werden.

19. Verfahren nach Anspruch 17, worin die Produkte durch ein Chromophor markiert werden.

20. Verfahren nach einem der Ansprüche 11 bis 16, worin das Nachweisen durch Hybridisieren des replizierten Produkts mit einer authentischen, gegebenenfalls markierten Zielsequenz durchgeführt wird.

21. Testkit, der geeignet ist, zum Nachweisen von wenigstens einer spezifischen RNA-Zielsequenz in einer Nukleinsäure enthaltenden Probe, wobei der Kit umfaßt:
(1) einen ersten Nukleinsäureprimer, umfassend:
(a) an seinem 5'-Ende eine erste Sequenz, von welcher Sequenz das Komplement ein Segment einer replizierbaren RNA ist, umfassend das 3'-Ende einer replizierbaren RNA, die zu einer durch RNA-abhängige RNA-Polymerase erfolgenden autokatalytischen Replikation fähig ist und
(b) an seinem 3'-Ende eine erste Sondensequenz, die fähig ist zum Hybridisieren an ein erstes Segment einer Ziel-RNA-Sequenz und zum Starten einer Extensionsreaktion unter Verwendung der Ziel-RNA-Sequenz als Matrize, um ein erstes Primerextensionsprodukt zu erzeugen, welches eine zu der Ziel-RNA-Sequenz komplementäre RNA-Sequenz umfaßt, und
(2) einen zweiten Nukleinsäureprimer, umfassend
(a) an seinem 5'-Ende eine zweite Sequenz, deren Komplement zu einer durch RNA-abhängige RNA-Polymerase erfolgenden katalytischen Replikation fähig ist und
(b) an seinem 3'-Ende eine zweite Sondensequenz, die fähig ist zum Hybridisieren an ein zweites Segment der komplementären Zielsequenz des ersten Primerextensionsprodukts, worin das zweite Segment gegenüberliegend zu dem ersten Segment in der Zielsequenz angeordnet ist und zum Starten einer Extensionsreaktion, unter Verwendung des ersten Extensionsprodukts als Matrize, um ein zweites Primerextensionsprodukt zu erzeugen, das die, die Matrizensequenz enthaltende autokatalytisch replizierbare RNA ist, mit der Maßgabe, daß die RNA, die aus der zweiten Sequenz, die mit ihrem 3'-Ende über ein einzelnes Phosphat an das 5'-Ende des Komplements der ersten Sequenz gebunden ist, besteht, autokatalytisch durch die Replikase replizierbar ist und mit der weiteren Maßgabe, daß eine RNA, die aus der autokatalytisch replizierbaren RNA dadurch hergestellt wird, daß darin zwischen dem 3'-Ende der zweiten Replikationsinitiationssequenz und dem 5'-Ende des Komplements der ersten Replikationsinitiationssequenz ein RNA-Segment insertiert wird, das ebenfalls autokatalytisch durch die Replikase replizierbar ist, und
(3) eine RNA-abhängige RNA-Polymerase.

## Revendications

1. Amorces d'acides nucléiques fonctionnant ensemble, utiles pour la formation d'un ARN réplicable de façon autocatalytique dans lequel est incorporée une séquence d'ARN cible, lesdites amorces fonctionnant ensemble comprenant
(1) une première amorce d'acide nucléique comprenant
(a) à son extrémité 5', une première séquence dont le complément est un segment d'un ARN réplicable qui comprend l'extrémité 3' d'un ARN réplicable capable d'une réplication autocatalytique dirigée par une ARN polymérase ARN-dépendante et
(b) à son extrémité 3', une première séquence sonde capable de s'hybrider à un premier segment d'une séquence d'ARN cible, et d'amorcer une réaction d'extension en utilisant la séquence d'ARN cible comme matrice, pour produire un produit d'extension de la première amorce qui comprend une séquence d'ARN complémentaire de la séquence d'ARN cible; et
(2) une seconde amorce d'acide nucléique comprenant
(a) à son extrémité 5', une seconde séquence dont le complément est capable d'une réplication catalytique dirigée par une ARN polymérase ARN-dépendante et
(b) à son extrémité 3', une seconde séquence sonde capable de s'hybrider à un second segment de ladite séquence cible complémentaire dudit produit d'extension de la première amorce, ledit second segment étant situé à l'opposé dudit premier segment dans ladite séquence cible, et d'amorcer une réaction d'extension en utilisant le premier produit d'extension comme matrice, pour produire un produit d'extension de la seconde amorce qui est un ARN réplicable de façon autocatalytique contenant ladite séquence cible; à condition que l'ARN qui est constitué de la seconde séquence reliée à son extrémité 3' par l'intermédiaire d'un seul phosphate à l'extrémité 5' du complément de la première séquence soit réplicable de façon autocatalytique par la réplicase, et à condition en outre qu'un ARN produit à partir dudit ARN réplicable de façon autocatalytique par insertion entre l'extrémité 3' de ladite seconde séquence d'amorçage de la réplication et l'extrémité 5' du complément de ladite première séquence d'amorçage de la réplication, soit un segment d'ARN qui est également réplicable de façon autocatalytique par la réplicase.

2. Amorces d'acide nucléique selon la revendication 1, dans lesquelles ladite ARN polymérase ARN-dépendante est la réplicase de Q-bêta.

3. Amorces d'acide nucléique selon la revendication 1 ou 2, dans lesquelles ladite séquence cible correspond à un segment d'ARN d'un virus du syndrome immunodéficitaire acquis.

4. Amorces d'acide nucléique selon la revendication 1 ou 2, dans lesquelles ladite séquence cible est un transcrit d'un gène défectif ou un transcrit défectif d'un gène normal.

5. Produit d'extension à brins séparés de la première amorce d'acide nucléique selon la revendication 1 hybridée à une seconde amorce d'acide nucléique selon la revendication 1.

6. Produit d'extension du complexe d'hybridation selon la revendication 5, dans lequel ledit produit d'extension, en présence d'une réplicase, réplique de façon autocatalytique la séquence cible.

7. Procédé de détection d'au moins une séquence cible d'ARN spécifique dans un échantillon contenant des acides nucléiques, ledit procédé comprenant la détection d'un produit d'extension réplicable, ledit produit étant le produit de l'extension d'une seconde amorce d'acide nucléique hybridée avec un brin séparé d'un premier produit d'extension qui contient une séquence d'une première amorce d'acide nucléique pouvant s'hybrider avec une séquence d'ARN cible, le dit produit d'extension réplicable fonctionnant comme molécule reporter pour ladite cible, ladite première amorce d'acide nucléique comprenant
(a) à son extrémité 5', une première séquence dont le complément est un segment d'un ARN réplicable qui comprend l'extrémité 3' d'un ARN réplicable capable d'une réplication autocatalytique dirigée par une ARN polymérase ARN-dépendante et
(b) à son extrémité 3', une première séquence sonde capable de s'hybrider à un premier segment d'une séquence d'ARN cible, et d'amorcer une réaction d'extension en utilisant la séquence d'ARN cible comme matrice, pour produire un produit d'extension de la première amorce qui comprend une séquence d'ARN complémentaire de la séquence d'ARN cible; et
et la seconde amorce d'acide nucléique comprenant
(a) à son extrémité 5', une seconde séquence dont le complément est capable d'une réplication catalytique dirigée par une ARN polymérase ARN-dépendante et
(b) à son extrémité 3', une seconde séquence sonde capable de s'hybrider à un second segment de ladite séquence cible complémentaire dudit produit d'extension de la première amorce, ledit second segment étant situé à l'opposé dudit premier segment dans ladite séquence cible, et d'amorcer une réaction d'extension en utilisant le produit d'extension de la première amorce comme matrice, pour produire un produit d'extension de la seconde amorce qui est un ARN réplicable de façon autocatalytique contenant ladite séquence cible; à condition que l'ARN qui est constitué de la seconde séquence reliée à son extrémité 3' par l'intermédiaire d'un seul phosphate à l'extrémité 5' du complément de ladite première séquence d'amorçage de la réplication soit un segment d'ARN qui est aussi réplicable de façon autocatalytique par la réplicase.

8. Procédé selon la revendication 7, comprenant l'étape supplémentaire selon laquelle on décèle le produit réplicable après avoir laissé ledit produit se répliquer.

9. Procédé selon la revendication 8, dans lequel ladite réplication s'effectue par mise en contact du produit réplicable avec une enzyme réplicase.

10. Procédé selon la revendication 9, dans lequel ladite réplicase est la réplicase de Q-bêta.

11. Procédé selon la revendication 7, comprenant les étapes selon lesquelles
on hybride avec ladite séquence d'ARN cible dans des conditions appropriées une première amorce d'acide nucléique, ladite première amorce d'acide nucléique comprenant à son extrémité 3' une première séquence sonde qui est complémentaire à un premier segment de ladite séquence cible, et à son extrémité 5', une première séquence dont le complément comprend un ARN réplicable capable d'une réplication par association avec une ARN polymérase ARN-dépendante appropriée,
on procède à l'extension de la chaîne de ladite première amorce d'acide nucléique hybridée,
on sépare les brins du produit d'extension obtenu,
on hybride ledit brin séparé avec une seconde amorce d'acide nucléique, ladite seconde amorce d'acide nucléique comprenant à son extrémité 3' une seconde séquence sonde qui est capable de s'hybrider à un second segment de ladite séquence cible complémentaire dudit brin séparé, ledit second segment étant situé à l'opposé dudit premier segment dans ladite séquence cible, et, à son extrémité 5', une longueur fonctionnelle d'une seconde séquence dont le complément est susceptible de se répliquer par association avec une ARN polymérase ARN-dépendante appropriée,
on procède à l'extension de la chaîne de ladite seconde amorce d'acide nucléique hybridée,
on permet au second produit d'extension ainsi produit de se répliquer, éventuellement après une séparation des brins, en mettant en contact ledit second produit d'extension avec une ARN polymérase ARN-dépendante, et
on décèle le produit de réplication.

12. Procédé selon la revendication 11, dans lequel ladite ARN polymérase ARN-dépendante est la réplicase de Q-bêta.

13. Procédé selon la revendication 11 ou 12, dans lequel on mesure d'une manière standardisée le produit décelé afin de mesurer la quantité de séquence cible contenue dans un échantillon d'acides nucléiques.

14. Procédé selon la revendication 11, 12 ou 13, dans lequel ladite séquence cible est contenue dans une séquence d'acide nucléique associée à une maladie ou un état génétique ou pathogène.

15. Procédé selon la revendication 14, dans lequel ladite séquence d'acide nucléique correspond à un segment d'ARN d'un virus du syndrome immunodéficitaire acquis.

16. Procédé selon la revendication 14, dans lequel ladite séquence d'acide nucléique est un transcrit d'un gène défectif ou un transcrit défectif d'un gène normal.

17. Procédé selon la revendication 11 ou 12, dans lequel lesdits produits décelés sont marqués avant la détection.

18. Procédé selon la revendication 17, dans lequel lesdits produits sont marqués par un marqueur radioactif.

19. Procédé selon la revendication 17, dans lequel lesdits produits sont marqués par un chromophore.

20. Procédé selon l'une quelconque des revendications 11 à 16, dans lequel on effectue ladite détection en hybridant le produit répliqué avec une séquence cible authentique, éventuellement marquée.

21. Trousse utile pour la détection d'au moins une séquence d'ARN cible spécifique dans un échantillon contenant des acides nucléiques, ladite trousse comprenant
(1) une première amorce d'acide nucléique comprenant
(a) à son extrémité 5', une première séquence dont le complément est un segment d'un ARN réplicable qui comprend l'extrémité 3' d'un ARN réplicable capable d'une réplication autocatalytique dirigée par une ARN polymérase ARN-dépendante et
(b) à son extrémité 3', une première séquence sonde capable de s'hybrider à un premier segment d'une séquence d'ARN cible, et d'amorcer une réaction d'extension en utilisant la séquence d'ARN cible comme matrice, pour produire un produit d'extension de la première amorce qui comprend une séquence d'ARN complémentaire de la séquence d'ARN cible; et
(2) une seconde amorce d'acide nucléique comprenant
(a) à son extrémité 5', une seconde séquence dont le complément est capable d'une réplication catalytique dirigée par une ARN polymérase ARN-dépendante et
(b) à son extrémité 3', une seconde séquence sonde capable de s'hybrider à un second segment de ladite séquence cible complémentaire dudit produit d'extension de la première amorce, ledit second segment étant situé à l'opposé dudit premier segment dans ladite séquence cible, et d'amorcer une réaction d'extension en utilisant le premier produit d'extension comme matrice, pour produire un produit d'extension de la seconde amorce qui est un ARN réplicable de façon autocatalytique contenant ladite séquence cible; à condition que l'ARN qui est constitué de la seconde séquence reliée à son extrémité 3' par l'intermédiaire d'un seul phosphate à l'extrémité 5' du complément de la première séquence soit réplicable de façon autocatalytique par la réplicase, et à condition en outre qu'un ARN produit à partir dudit ARN réplicable de façon autocatalytique par insertion entre l'extrémité 3' de ladite seconde séquence d'amorçage de la réplication et l'extrémité 5' du complément de ladite première séquence d'amorçage de la réplication, soit un segment d'ARN qui est également réplicable de façon autocatalytique par la réplicase, et
(3) une ARN polymérase ARN-dépendante.
